(19) Europäisches Patentamt
European Patent Office
Office européen des brevets

(11) EP 0 780 112 A1

# (12) EUROPÄISCHE PATENTANMELDUNG

(43) Veröffentlichungstag:
**25.06.1997 Patentblatt 1997/26**

(51) Int. Cl.[6]: **A61K 7/00**, B01F 17/00

(21) Anmeldenummer: **96119424.8**

(22) Anmeldetag: **04.12.1996**

(84) Benannte Vertragsstaaten:
**AT BE CH DE ES FR GB IT LI NL**

(30) Priorität: **21.12.1995 DE 19548013**

(71) Anmelder: **Beiersdorf Aktiengesellschaft**
**D-20245 Hamburg (DE)**

(72) Erfinder:
- **Gers-Barlag, Heinrich, Dr.**
  **25495 Kummerfeld (DE)**
- **Gohla, Sven, Dr.**
  **21077 Hamburg (DE)**
- **Nielsen, Jens**
  **24558 Henstedt-Ulzburg (DE)**
- **Müller, Anja**
  **20253 Hamburg (DE)**

## (54) Verfahren zur Herstellung von O/W- oder O/W/O-Emulsionen sowie nach solchen Verfahren erhältliche O/W- und O/W/O-Emulsionen

(57) O/W-Emulsionen, insbesondere O/W-Mikroemulsionen, oder O/W/O-Emulsionen oder O/W/O'-Emulsionen, enthaltend mindestens einen Emulgator (Emulgator A), gewählt aus der Gruppe der Emulgatoren mit folgenden Eigenschaften

- ihre Lipophilie ist entweder abhängig vorn pH-Wert, dergestalt daß durch Erhöhung oder Senkung des pH-Wertes die Lipophilie zunimmt oder abnimmt, wobei unerheblich ist, welche der beiden Möglichkeiten der Änderung der Lipophilie durch die Erhöhung bzw. Senkung des pH-Wertes bewirkt wird, und/oder
- ihre Lipophilie ist abhängig von der Temperatur, dergestalt, daß die Lipophilie mit steigender Temperatur zunimmt und deren Hydrophilie mit sinkender Temperatur zunimmt.

EP 0 780 112 A1

Printed by Rank Xerox (UK) Business Services
2.14.8/3.4

## Beschreibung

Die vorliegende Erfindung betrifft stabile Emulsionen vom O/W- bzw. O/W/O- bzw. O/W/O'-Typ, Verfahren zu ihrer Herstellung sowie ihre Verwendung für kosmetische und medizinische Zwecke.

Unter kosmetischer Hautpflege ist in erster Linie zu verstehen, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ein Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Medizinische topische Zubereitungen enthalten in der Regel Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Es ist bekannt, daß sich multiple Emulsionen - unter anderem - durch eine besonders feine Emulsionstextur auszeichnen können. Diese Eigenschaft eignet sie hervorragend als Basis sowohl für kosmetische wie für medizinische topische Zubereitungen. Wo allerdings Kosmetika nur äußerlich angewandt werden, sind bei medizinischer Verwendung von Emulsionen alle üblichen Applikationsarten, z.B. orale Verabreichungsformen, denkbar.

In einfachen Emulsionen liegen in der einen Phase feindisperse, von einer Emulgatorhülle umschlossene Tröpfchen der zweiten Phase (Wassertröpfchen in W/O- oder Lipidvesikel in O/W-Emulsionen) vor. In einer multiplen Emulsion (zweiten Grades) hingegen sind in solchen Tröpfchen feiner disperse Tröpfchen der ersten Phase emulgiert. Auch in diesen Tröpfchen wiederum können noch feiner disperse Tröpfchen vorliegen (multiple Emulsion dritten Grades) und so fort.

So wie man also bei den einfachen Emulsionen von W/O- oder O/W-Emulsionen spricht (Wasser-in-Oel oder Oel-in-Wasser), gibt es bei multiplen Emulsionen W/O/W-, O/W/O-, O/W/O/W-, W/O/W/O-Emulsionen und so fort.

Multiple Emulsionen, bei welchen die jeweiligen inneren und äußeren Wasserphasen oder inneren und äußeren Ölphasen unterschiedlich geartet sind (also z.B. W/O/W- und O/W/O' -Emulsionen), sind der Präparation durch Zweitopfverfahren zugängig. Solche Emulsionen, in welchen die inneren und äußeren Wasser- bzw. Ölphasen nicht unterschiedlich geartet sind, sind sowohl durch Ein- als auch durch Zweitopfverfahren erhältlich.

In Figur 1 wird eine O/W/O-Emulsion schematisch dargestellt, wobei die weißen Flächen die äußere und innere Ölphase und die schraffierten Flächen die Wasserphase bedeuten. Offengelassen wurde, ob sich die Zusammensetzungen der äußeren und inneren Ölphase voneinander unterscheiden, ob also eine O/W/O- oder eine O/W/O'-Emulsion vorliegt.

Die multiplen Emulsionen zweiten Grades werden gelegentlich als "bimultiple Systeme", solche dritten Grades als "trimultiple Systeme" usw., bezeichnet (W.Seifriz, Studies in Emulsions, J.Phys.Chem., 29 (1925) 738 - 749).

Verfahren zur Herstellung multipler Emulsionen sind dem Fachmann an sich geläufig. So gibt es Zweitopfverfahren, in welchen eine einfache Emulsion (z.B. eine W/O-Emulsion) vorgelegt und durch Zugabe einer weiteren Phase (z.B. eine Wasserphase) mit einem entsprechenden Emulgator (z.B. ein O/W-Emulgator) in eine multiple Emulsion (z.B. eine W/O/W- Emulsion) überführt wird.

Eine zweites bekanntes Verfahren besteht darin, Emulgatorgemische mit einer Ölphase und einer Wasserphase in einem Eintopfverfahren in eine multiple W/O/W-Emulsion zu überführen. Die Emulgatoren werden in der Ölphase gelöst und mit der Wasserphase vereinigt. Voraussetzung für ein solches Verfahren ist, daß die HLB-Werte (HLB = Hydrophil-Lipophil-Balance) der eingesetzten einzelnen Emulgatoren sich deutlich voneinander unterscheiden.

Die Definition für den HLB-Wert ist für Polyolfettsäureester gegeben durch die Formel I

$$HLB = 20 * ( 1 - S/A)$$

Für eine Gruppe von Emulgatoren, deren hydrophiler Anteil nur aus Ethylenoxideinheiten besteht, gilt die Formel II

$$HLB = E/5$$

wobei S = Verseifungszahl des Esters,
A = Säurezahl der zurückgewonnen Säure
E = Massenanteil Ethylenoxid (in %) am Gesamtmolekül

bedeuten.

Emulgatoren mit HLB-Werten von 6-8 sind im allgemeinen W/O-Emulgatoren, solche mit HLB-Werten von 8-18 sind im allgemeinen O/W-Emulgatoren.

Literatur: "Kosmetik - Entwicklung, Herstellung und Anwendung kosmetischer Mittel"; W.Umbach (Hrsg.), Georg Thieme Verlag 1988.

Hydrophile Emulgatoren (mit hohen HLB-Werten) sind in der Regel O/W-Emulgatoren. Demgemäß sind hydrophobe oder lipophile Emulgatoren (mit niedrigen HLB-Werten) in der Regel W/O-Emulgatoren.

Die US-Patentschrift 4,931,210 beschreibt ein Verfahren zur Herstellung von W/O/W-Emulsion, wobei als Emulgatoren Polyglycerinpolyricinoleate verwendet werden.

Obwohl also multiple Emulsionen an sich bekannt sind und es durchaus einfache Verfahren zu ihrer Herstellung gibt, hat es dennoch bis heute an solchen Systemen gemangelt, welche mikroskopisch über längere Lagerzeiten (beispielsweise über mehrere Jahre) bzw. in einem breiten Temperaturbereich (beispielsweise von -10° C bis +50° C) bzw. gegenüber extremen Temperaturschwankungen stabil (schaukelstabil, beipielsweise von -15 bis +50° C) sind. Dies soll bedeuten, daß sich die multiplen Emulsionen des Standes der Technik mit der Zeit in einfache W/O- oder O/W-Emulsionen umwandeln, also eine im Sinne der Multiplizität geringe Lagerungsstabilität aufweisen. Dies ist insbesondere nachteilig, als diese Umwandlungsprodukte in der Regel eine äußerst inhomogene Tröpfchengrößenverteilung haben.

Bestenfalls sind solche Umwandlungsprodukte unschön oder aus kosmetischer Sicht unelegant. Oft ist jedoch mit der inhomogenen Größenverteilung der Tröpfchen auch mangelnde makroskopische Stabilität verbunden, also die Stabilität gegen die Zersetzung in getrennte Phasen.

Auch in dieser Hinsicht waren die herkömmlichen multiplen Emulsionen stets entweder unzureichend stabil, oder aber die Übertragung vom Labormaßstab auf die großtechnische Produktion war nicht durchführbar.

Insbesondere nachteilig ist der Mangel an O/W/O-Emulsionen, da im druckschriftlichen Stande der Technik zumeist W/O/W-Emulsionen beschrieben werden. Kosmetische oder dermatologische O/W/O-Emulsionen sind derzeit eher Laborspezialitäten als auf dem Markte erhältliche Produkte.

Die Tröpfchendurchmesser der gewöhnlichen „einfachen", also nichtmultiplen Emulsionen liegen im Bereich von ca 1 μm bis ca. 50 μm. Solche „Makroemulsionen" sind, ohne weitere färbende Zusätze, milchigweißgefärbt und opak. Feinere „Makroemulsionen", deren Tröpfchendurchmesser im Bereich von ca. $10^{-1}$ μm bis ca. 1 μm liegen, sind, wiederum ohne färbende Zusätze, bläulichweißgefärbt und opak. Solche „Makroemulsionen" haben für gewöhnlich hohe Viskosität.

Mizellaren und molekularen Lösungen mit Partikeldurchmessern kleiner als ca. $10^{-2}$ μm, die allerdings nicht mehr als echte Emulsionen aufzufassen sind, ist vorbehalten, klar und transparent zu erscheinen.

Der Tröpfchendurchmesser von Mikroemulsionen dagegen liegt im Bereich von etwa $10^{-2}$ μm bis etwa $10^{-1}$ μm. Mikroemulsionen sind transluzent und meist niedrigviskos. Die Viskosität vieler Mikroemulsionen vom O/W-Typ ist vergleichbar mit der des Wassers.

Vorteil von Mikroemulsionen ist, daß in der dispersen Phase Wirkstoffe wesentlich feiner dispers vorliegen können als in der dispersen Phase von „Makroemulsionen". Ein weiterer Vorteil ist, daß sie aufgrund ihrer niedrigen Viskosität versprühbar sind. Werden Mikroemulsionen als Kosmetika verwendet, zeichnen sich entsprechende Produkte durch hohe kosmetische Eleganz aus.

Nachteilig an den Mikroemulsionen des Standes der Technik ist, daß stets ein hoher Gehalt an einem oder mehreren Emulgatoren eingesetzt werden muß, da die geringe Tröpfchengröße eine hohe Grenzfläche zwischen den Phasen bedingt, welche in der Regel durch Emulgatoren stabilisiert werden muß.

Wegen der guten Versrpühbarkeit eigenen sich Mikroemulsionen grundsätzlich auch für andere kosmetische dermatologische Anwendungen, beispielsweise Desodorantien, so daß die vorliegende Erfindung in einer besonderen Ausführungsform Mikroemulsionen als Grundlage für kosmetische Desodorantien betrifft.

Kosmetische Desodorantien dienen dazu, Körpergeruch zu beseitigen, der entsteht, wenn der an sich geruchlose frische Schweiß durch Mikroorganismen zersetzt wird. Den üblichen kosmetischen Desodorantien liegen unterschiedliche Wirkprinzipien zugrunde.

In sogenannten Antitranspirantien kann durch Adstringentien - vorwiegend Aluminiumsalze wie Aluminiumhydroxychlorid (Aluchlorhydrat) - die Bildung des Schweißes reduziert werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. Der Schweißfluß selbst wird dadurch nicht beeinflußt, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich.

Desodorantien sollen folgende Bedingungen erfüllen:

1) Sie sollen eine zuverlässige Desodorierung bewirken.
2) Die natürlichen biologischen Vorgänge der Haut dürfen nicht durch die Desodorantien beeinträchtigt werden.
3) Die Desodorantien müssen bei Überdosierung oder sonstiger nicht bestimmungsgemäßer Anwendung

unschädlich sein.

4) Sie sollen sich nach wiederholter Anwendung nicht auf der Haut anreichern.

5) Sie sollen sich gut in übliche kosmetische Formulierungen einarbeiten lassen.

Bekannt und gebräuchlich sind sowohl flüssige Desodorantien, beispielsweise Aerosolsprays, Roll-ons und dergleichen als auch feste Zubereitungen, beispielsweise Deo-Stifte ("Sticks"), Puder, Pudersprays, Intimreinigungsmittel usw.

Die herkömmlichen Zubereitungen in Emulsionsform sind zwar im allgemeinen gut wirksam, zeichnen sich aber nur bedingt durch Pflegewirkung aus. Auch die Verwendung von Mikroemulsionen als Grundlage für desodorierende oder antitranspirant wirkende Zubereitungen sind bekannt. Deren relativ hoher Gehalt an Emulgatoren, mit den geschilderten Nachteilen, war bisher ein Übelstand, dem es abzuhelfen galt.

Eine weitere Aufgabe der vorliegenden Erfindung war es also, Zubereitungen zu entwickeln, welche als Grundlage für kosmetische Desodorantien bzw. Antitranspirantien geeignet sind, und die Nachteile des Standes der Technik nicht aufweisen.

Weiterhin war es eine Aufgabe der Erfindung, kosmetische Grundlagen für kosmetische Desodorantien zu entwikkeln, die sich durch gute Hautverträglichkeit auszeichnen.

Ferner war eine Aufgabe der vorliegenden Erfindung, Produkte auf der Basis von Mikroemulsionen oder O/W/O-Emulsionen mit einer möglichst breiten Anwendungsvielfalt zur Verfügung zu stellen. Beispielsweise sollten Grundlagen für Zubereitungsformen wie Reinigungsemulsionen, Gesichts- und Körperpflegezubereitungen, aber auch ausgesprochen medizinisch-pharmazeutische Darreichungsformen geschaffen werden, zum Beispiel Zubereitungen gegen Akne und andere krankhafte Hauterscheinungen. Schließlich sollte auch grundsätzlich der Weg zu innerlich anwendbaren Emulsionen, beispielsweise für die parenterale Ernährung durch die vorliegende Erfindung eröffnet werden.

Eine besondere Aufgabe der vorliegenden Erfindung war es, feindisperse Zubereitungen vom Typ Öl-in-Wasser oder multiple Emulsionen vom Typ O/W/O mit einem möglichst niedrigen Emulgatorgehalt zur Verfügung zu stellen, welche nicht die Nachteile des Standes der Technik aufweisen und welche für verschiedenste kosmetische und/oder dermatologische Anwendungen, beispielsweise die vorab beschreibenen Verwendungen finden können. Eine weitere Aufgabe der Erfindung war, das begrenzte Angebot an feindispersen Zubereitungen vom Typ Öl-in-Wasser des Standes der Technik zu bereichem.

Es ist bekannt, daß hydrophile Emulgatoren bei steigender Temperatur ihr Löslichkeitsverhalten von wasserlöslich zu fettlöslich ändern. Der Temperaturbereich, in dem die Emulgatoren ihre Löslichkeit geändert haben, wird Phaseninversionstemperaturbereich (PIT) genannt.

S.Matsumoto (Journal of Colloid and Interface Science, Vol. 94, No.2, 1983) beschreibt, daß die Entwicklung einer W/O/W-Emulsion einer Phaseninversion konzentrierter W/O-Emulsionen, welche durch Span 80, einen ausgeprägten W/O-Emulgator, stabilisiert sind, vorausgeht. Matsumoto geht dabei von einem extrem unpolaren Öl, nämlich flüssigem Paraffin aus. Darüberhinaus sei eine gewisse Menge hydrophiler Emulgatoren zur Entwicklung einer W/O/W-Emulsion aus einer W/O-Emulsion nötig.

T.J.Lin, H.Kurihara und H.Ohta (Journal of the Society of Cosmetic Chemists 26, S. 121 - 139, März 1975 zeigen bei unpolaren Ölen, daß im Bereich der PIT extrem instabile multiple Emulsionen vorliegen können.

Auch Mikroemulsionen sind über die Phaseninversionstechnologie der Präparation zugängig. Auf solche Weise hergestellte Mikroemulsionen des Standes der Technik haben allerdings den Nachteil, daß erstens die Tröpfchengröße immer noch recht hoch ist und zweitens immer noch ein hoher Anteil an einem oder mehreren Emulgatoren nötig ist.

Weiterhin ist nachteilig, daß auf solche Weise hergestellte Mikroemulsionen zwar bei hoher Temperatur, also beispielsweise im PIT, praktisch transparent sind, aber beim Absinken auf Raumtemperatur wieder transluzent oder opak werden.

Auch diesen Übelständen galt es also, abzuhelfen.

Aufgabe der vorliegenden Erfindung war also, stabile Mikroemulsionen vom Typ O/W bzw. stabile multiple Emulsionen vom Typ O/W/O bzw. O/W/O' zur Verfügung zu stellen und die Nachteile der Zubereitungen des Standes der Technik bzw. deren Herstellungsverfahren zu beseitigen.

Es hat sich überraschenderweise gezeigt, und darin liegt die Lösung der Aufgaben begründet, daß O/W-Emulsionen, insbesondere O/W-Mikroemulsionen, oder O/W/O-Emulsionen oder O/W/O'-Emulsionen, enthaltend

- eine Wasserphase,
- gegebenenfalls übliche, in Wasser lösliche oder dispergierbare Substanzen,
- eine Ölphase,
- mindestens einen Emulgator (Emulgator A), gewählt aus der Gruppe der Emulgatoren mit folgenden Eigenschaften
    - ihre Lipophilie ist entweder abhängig vom pH-Wert, dergestalt daß durch Erhöhung oder Senkung des pH-Wertes die Lipophilie zunimmt oder abnimmt, wobei unerheblich ist, welche der beiden Möglichkeiten der

Änderung der Lipophilie durch die Erhöhung bzw. Senkung des pH-Wertes bewirkt wird, und/oder

- ihre Lipophilie ist abhängig von der Temperatur, dergestalt, daß die Lipophilie mit steigender Temperatur zunimmt und deren Hydrophilie mit sinkender Temperatur zunimmt,

- ferner gegebenenfalls weitere in der Ölphase lösliche oder dispergierbare Substanzen, darunter bevorzugt solche, gewählt aus der Gruppe der nicht unter die Definition des Emulgators A fallende Emulgatoren, insbesondere solche, die vorwiegend als W/O-Emulgatoren wirken,

den Nachteilen des Standes der Technik abhelfen.

Als vorteilhafte Verkörperung der vorliegenden Erfindung wird ebenfalls angesehen ein Verfahren zur Herstellung von O/W-Emulsionen, insbesondere O/W-Mikroemulsionen, oder O/W/O-Emulsionen oder O/W/O'-Emulsionen, dadurch gekennzeichnet, daß eine Wasserphase, gegebenenfalls übliche, in Wasser lösliche oder dispergierbare Substanzen, eine Ölphase sowie

- mindestens ein Emulgator (Emulgator A), gewählt aus der Gruppe der O/W-Emulgatoren mit folgenden Eigenschaften

  - ihre Lipophilie ist abhängig vom pH-Wert, dergestalt daß durch Erhöhung oder Senkung des pH-Wertes die Lipophilie zunimmt oder abnimmt, wobei unerheblich ist, welche der beiden Möglichkeiten der Änderung der Lipophilie durch die Erhöhung bzw. Senkung des pH-Wertes bewirkt wird, und
  - ihre Lipophilie ist gegebenenfalls zusätzlich abhängig von der Temperatur, dergestalt, daß die Lipophilie mit steigender Temperatur zunimmt und deren Hydrophilie mit sinkender Temperatur zunimmt,

ferner gegebenenfalls weitere in der Ölphase lösliche oder dispergierbare Substanzen, darunter bevorzugt solche, gewählt aus der Gruppe der nicht unter die Definition des Emulgators A fallende Emulgatoren,
zusammengegeben werden und unter Agitation ein Gemisch gebildet wird, dergestalt, daß

- dieses Gemisch durch geeignete Wahl der Parameter, gewählt aus der Gruppe pH-Wert, Temperatur und der Konzentration bzw. Konzentrationen mindestens eines der gewählten Emulgatoren, in den Phaseninversionsbereich gebracht wird, in welchem sich W/O-Emulsionen in O/W-Emulsionen umwandeln,
- durch Variation mindestens eines Parameters, gewählt aus der Gruppe pH-Wert, Temperatur und der Konzentration bzw. Konzentrationen mindestens eines der gewählten Emulgatoren, die gebildete W/O-Emulsion aus dem Phaseninversionsbereich heraus gebracht wird, in welchem sich eine gebildete W/O-Emulsion in eine O/W-Emulsion umwandelt, wodurch eine O/W-Emulsion oder O/W-Mikroemulsion erzeugt wird.
- gegebenenfalls durch geeignete Wahl der Rahmenbedingungen eine weitere Phaseninversion zur O/W/O-Emulsion eingeleitet wird,
- das Gemisch gegebenenfalls weiteren Aufbereitungsschritten, insbesondere einem oder mehreren Homogenisierungsschritten unterworfen wird.

Erfindungsgemäß gleichermaßen vorteilhaft sind Verfahren, bei welchen die Variation des Parameters oder der Parameter darin besteht, daß

(a) bei vorgegebenem pH-Wert und vorgegebener Konzentration des Emulgators A bzw. der Vielzahl an Emulgatoren A die Temperatur des Gemisches sowie gegebenenfalls zusätzlich die Konzentration mindestens eines weiteren Emulgators A variiert wird, daß
(b) bei vorgegebener Temperatur und vorgegebener Konzentration des Emulgators A bzw. der Vielzahl an Emulgatoren A der pH-Wert des Gemisches sowie gegebenenfalls zusätzlich die Konzentration mindestens eines weiteren Emulgators A variiert wird, daß
(c) bei vorgegebener Temperatur und vorgegebenem pH-Wert die Konzentration mindestens eines Emulgators A sowie gegebenenfalls zusätzlich die Konzentration mindestens eines weiteren Emulgators A variiert wird, daß
(d) bei vorgegebenem pH-Wert die Temperatur des Gemisches sowie zusätzlich die Konzentration mindestens eines Emulgators A sowie gegebenenfalls zusätzlich die Konzentration mindestens eines weiteren Emulgators A variiert werden, daß
(e) bei vorgegebener Temperatur des Gemisches der pH-Wert des Gemisches sowie zusätzlich die Konzentration mindestens eines Emulgators A sowie gegebenenfalls zusätzlich die Konzentration mindestens eines weiteren Emulgators A variiert werden, daß
(f) bei vorgegebener Konzentration mindestens eines Emulgators A der pH-Wert sowie zusätzlich die Temperatur des Gemisches sowie gegebenenfalls zusätzlich die Konzentration mindestens eines weiteren Emulgators A variiert werden.

Sofern die Phaseninversion im wesentlichen durch Variation der Temperatur eingeleitet wird, sind O/W-Emulsionen, insbesondere O/W-Mikroemulsionen erhältlich, wobei die Größe der Öltröpfchen im wesentlichen durch die Konzentration des oder der eingesetzten Emulgatoren bestimmt wird, dergestalt, daß eine höhere Emulgatorkonzentration kleinere Tröpfchen bewirkt und geringere Emulgatorkonzentration zu größeren Tröpfchen führt. Es ist, wenn die Phaseninversion im wesentlichen durch Variation der Temperatur eingeleitet wird, durchaus vorteilhaft, auf weitere, nicht unter die Definition des Emulgators A fallende, weitere Emulgatoren, namentlich W/O-Emulgatoren, zu verzichten.

Sofern die Phaseninversion im wesentlichen durch Variation des pH-Wertes eingeleitet wird, sind O/W-Emulsionen, insbesondere O/W-Mikroemulsionen, aber auch O/W/O-Emulsionen erhältlich. Es ist, wenn die Phaseninversion im wesentlichen durch Variation des pH-Wertes eingeleitet wird, durchaus vorteilhaft, einen oder mehrere weitere, nicht unter die Definition des Emulgators A fallende, weitere Emulgatoren, namentlich W/O-Emulgatoren, einzusetzen.

Erfindungsgemäß können O/W/O-Emulsionen erhalten werden, wenn der Ölphasenanteil höher als etwa 15 Gew.-%, insbesondere höher als etwa 20 Gew.-%, bezogen auf das Gesamtgewicht der Zubereitung liegt, mehr als etwa 5 Gew.-%, insbesondere etwa 5 - 10 Gew.-% eines zusätzlichen, nicht unter die Definition des Emulgators A fallenden W/O-Emulgators vorliegen und/oder wenn die Ölphase einen niedrigen Anteil an polaren Ölen aufweist.

Erfindungsgemäß können O/W-Mikroemulsionen erhalten werden, wenn der Ölphasenanteil unter etwa 20 Gew.-%, insbesondere unter etwa 15 Gew.-%, bezogen auf das Gesamtgewicht der Zubereitung liegt, weniger als etwa 5 Gew.-% eines zusätzlichen, nicht unter die Definition des Emulgators A fallenden W/O-Emulgators vorliegen und/oder wenn die Ölphase einen hohen Anteil an polaren Ölen aufweist.

Erfindungsgemäß können O/W-Emulsionen („Makroemulsionen“) erhalten werden, wenn weniger als etwa 5 Gew.-% eines zusätzlichen nicht unter die Definition des Emulgators A fallenden W/O-Emulgators und mehr als etwa 20 Gew.-% einer polaren Ölphase vorliegen. Vorteilhaft können zusätzliche Gelbildner (z.B. Carbopole, Xanthangummi, Cellulosederivate) eingesetzt werden.

Es ist dabei im Einzelfalle möglich, daß die vorgenannten Konzentrationsgrenzen leicht über- oder unterschritten werden und dennoch die betreffenden Emulsionstypen erhalten werden. Dies kommt angesichts der breit streuenden Vielfalt an geeigneten Emulgatoren und Ölbestandteilen für den Fachmann nicht unerwartet, so daß er weiß, daß bei solchen Über- oder Unterschreitungen der Boden der vorliegenden Erfindung nicht verlassen wird.

Der Phaseninversionsbereich läßt sich mathematisch darstellen als Punktmenge innerhalb des geradlinigen Koordinatensystems $\Sigma$, welches durch die Größen Temperatur, pH-Wert und Konzentration eines geeigneten Emulgators bzw. eines Emulgatorengemisches in der Zubereitung gebildet wird, gemäß:

$$\Sigma = \{O, \theta, a, m\},$$

mit O - Koordinatenursprung
$\theta$ - Temperatur
a - pH-Wert
m - Konzentration

Dabei muß genaugenommen natürlich in einem mehrkomponentigen Emulgatorensystem der Beitrag $m_i$ jedes einzelnen Emulgators zur Gesamtfunktion berücksichtigt werden, was bei einem i-komponentigen Emulgatorensystem zur Beziehung

$$\Sigma = \{O, \theta, a, m_1, m_2, ..., m_i\} \text{ führt.}$$

Der Phaseninversionsbereich $\Phi$ stellt dabei im mathematischen Sinne ein zusammenhängendes Gebiet oder eine Vielzahl zusammenhängender Gebiete innerhalb des Koordinatensystems $\Sigma$ dar. $\Phi$ repräsentiert die Gesamtmenge der Koordinatenpunkte $K(\theta, a, m_1, m_2, ..., m_i)$, welche erfindungsgemäße Gemische aus Wasser- und Ölphase, i erfindungsgemäßen Emulgatoren der Konzentration $m_i$ bei der Temperatur $\theta$ und dem pH-Wert a bestimmen, und für welche gilt, daß beim Übergang von einer Koordinate $K_1 \notin \Phi$ zu einer Koordinate $K_2 \in \Phi$ Phaseninversion eintritt, wie in Fig. 2 beschrieben. Als variable Koordinaten in Fig.2 wurden Temperatur und pH-Wert eines gegebenen Gemisches unter Konstanthaltung der Konzentration von $m_1$ - $m_i$ angegeben. Beim Übergang von $K_1$ nach $K_2$ wird lediglich die Temperatur erhöht.

Unter den erfindungsgemäßen Bedingungen ist dieser Prozeß nicht reversibel, d.h., kehrt das System von der Koordinate $K_2 \in \Phi$ wieder zur Koordinate $K_1 \notin \Phi$ zurück, können erfindungsgemäß andere Emulsionstypen erhalten werden, als der Stand der Technik hätte erwarten lassen. Beispielsweise wird durch Erhöhen der Temperatur eines erfindungsgemäßen Gemisches aus Wasser- und Ölphase, i erfindungsgemäßen Emulgatoren der Konzentrationen $m_i$, wobei der pH-Wert a konstant bleibt, ausgehend von einer Temperatur welche für Phaseninversion zu niedrig ist (Bedingungen also, wo eine herkömmliche O/W-Emulsion vorläge, welche auch beim Abkühlen auf Raumtemperatur eine solche bliebe), erwärmt wird, so daß Phaseninversion eintritt, wird nach Abkühlen, z.B. auf Raumtemperatur, keine herkömmliche O/W-Emulsion erhalten, sondern eine erfindungsgemäße O/W-Mikroemulsion. Oder, mutatis mutandis:

eine erfindungsgemäße O/W/O-Emulsion.

Unerheblich ist dabei, ob der Phaseninversionsbereich eines gegebenen Systems ein einziges zusammenhängendes (i + 2)-dimensionalen Gebiet darstellt oder aus mehreren zusammenhängenden, aber voneinander getrennten solchen Gebieten besteht, also mehreren Phaseninversionsbereichen eines gegebenen Systems entsprechend. Im Rahmen der hiermit vorgelegten Offenbarung wird daher stets verallgemeinernd von einem Phaseninversionsbereich gesprochen, auch bei Vorliegen zweier oder mehrerer voneinander getrennter solcher Bereiche.

Die Praxis der Herstellung einer erfindungsgemäßen Emulsion besteht vorteilhaft darin, nach Auswahl geeigneter Rohstoffe, d.h., Wasser- und Ölphase, ein oder mehrere Emulgatoren vom Typ A, letzterer oder letztere vorliegend in Konzentrationen, bei welchen Phaseninversion für das gegebene Gemisch möglich ist, und gegebenenfalls weitere Substanzen, die Einzelkomponenten unter Agitation auf eine Temperatur zu erwärmen, bei welcher Phaseninversion für das gegebene Gemisch möglich ist, und durch Erhöhung oder Erniedrigung des pH-Wertes des Gemisches Phaseninversion herbeizuführen, hernach unter fortwährender Agitation das Gemisch auf Raumtemperatur abkühlen zu lassen. Ein oder mehrere zwischengeschaltete Homogenisierungsschritte sind vorteilhaft, aber nicht zwingend notwendig.

Eine weitere vorteilhafte Ausführungsform des erfindungsgemäßen Verfahrens besteht darin, nach der Auswahl geeigneter Rohstoffe, d.h., Wasser- und Ölphase, ein oder mehrere Emulgatoren vom Typ A letzterer oder letztere vorliegend in Konzentrationen, bei welchen Phaseninversion für das gegebene Gemisch möglich ist, und gegebenenfalls weitere Substanzen, die Einzelkomponenten unter Agitation auf einen pH-Wert zu bringen, bei welchem Phaseninversion für das gegebene Gemisch möglich ist, und durch Erhöhung der Temperatur des Gemisches Phaseninversion herbeizuführen, hernach unter fortwährender Agitation das Gemisch auf Raumtemperatur ankühlen zu lassen. Ein oder mehrere zwischengeschaltete Homogenisierungsschritte sind vorteilhaft, aber nicht zwingend notwendig.

Eine dritte vorteilhafte Ausführungsform des Erfindungsgemäßen Verfahrens besteht darin, nach der Auswahl geeigneter Rohstoffe, d.h., Wasser- und Ölphase, ein oder mehrere Emulgatoren vom Typ A und gegebenenfalls weitere Substanzen, die Einzelkomponenten unter Agitation auf einen pH-Wert und eine Temperatur zu bringen, bei welchen Phaseninversion für das gegebene Gemisch möglich ist, und durch Zugabe des Emulgators A oder der Emulgatoren A zum Gemisch Phaseninversion herbeizuführen, hernach unter fortwährender Agitation das Gemisch auf Raumtemperatur ankühlen zu lassen. Ein oder mehrere zwischengeschaltete Homogenisierungsschritte sind vorteilhaft, aber nicht zwingend notwendig.

In der Praxis ist möglich und gegebenenfalls sogar vorteilhaft, bei der Herstellung einer erfindungsgemäßen Emulsion den Temperaturbereich, der dem Phaseninversionsbereich zugeordnet werden kann auch zu übersteigen, da beim Abkühlen auf Raumtemperatur dieser Bereich dann zwangsläufig durchlaufen wird.

Die Emulgatoren A werden bevorzugt gewählt aus der Gruppe der Emulgatoren, welche gute Protonendonoren bzw. Protonenakzeptoren darstellen, wobei gewährleistet sein muß, daß ihre Lipophilie abhängig ist vom pH-Wert, dergestalt daß durch Erhöhung oder Senkung des pH-Wertes die Lipophilie zunimmt oder abnimmt, wobei grundsätzlich unerheblich ist, welche der beiden Möglichkeiten der Änderung der Lipophilie durch die Erhöhung bzw. Senkung des pH-Wertes bewirkt wird, und gegebenenfalls ihre Lipophilie zusätzlich abhängig ist von der Temperatur, dergestalt, daß ihre Lipophilie mit steigender Temperatur zunimmt und ihre Hydrophilie mit sinkender Temperatur zunimmt.

Die Lipidphase kann vorteilhaft gewählt werden aus folgender Substanzgruppe:

- Öle, wie Triglyceride der Caprin- oder der Caprylsäure
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;

Ganz besonders vorteilhaft kann die Lipidphase gewählt werden aus der Gruppe der Silikonöle wie Dimethylpolysiloxane, Diethylpolysiloxane, Diphenylpolysiloxane sowie Mischformen daraus

Die wäßrige Phase der erfindungsgemäßen Zubereitungen enthält gegebenenfalls vorteilhaft

- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Vorteilhaft werden die Emulgatoren des Typs A gewählt aus der Gruppe der Sorbitanester und Saccharoseester, insbesondere der verzweigten und unverzweigten Alkylester und Alkenylester mit Kohlenstoffketten von 4 - 24 Kohlenstoffatomen, bevorzugt Sorbitanstearat, Sorbitanoleat, Glycerylsorbitanstearat, Saccharosemonostearat, Saccharosemonolaurat, Saccharosepalmitat.

Vorteilhaft können die Emulgatoren des Typs A gewählt aus der Gruppe der Monoglycerin-monocarbonsäuremonoester gewählt werden, insbesondere solche, welche durch die Strukturen

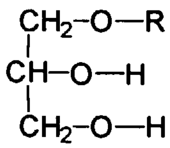

bzw.

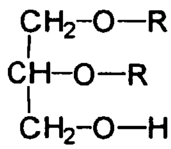

gekennzeichnet sind, wobei R einen verzweigten oder unverzweigten Acylrest mit 6 - 14 Kohlenstoffatomen darstellt. Vorteilhaft wird R gewählt aus der Gruppe der unverzweigten Acylreste. Die diesen Estern zugrundeliegenden Fettsäuren bzw. Monocarbonsäuren sind die

Hexansäure (Capronsäure)
(R = $-C(O)-C_5H_{11}$),
Heptansäure (Önanthsäure)
(R = $-C(O)-C_6H_{13}$),
Octansäure (Caprylsäure)
(R = $-C(O)-C_7H_{15}$),
Nonansäure (Pelargonsäure)
(R = $-C(O)-C_8H_{17}$),
Decansäure (Caprinsäure)
(R = $-C(O)-C_9H_{19}$),
Undecansäure
(R = $-C(O)-C_{10}H_{21}$),
Dodecansäure (Laurinsäure)
(R = $-C(O)-C_{11}H_{23}$),
Tridecansäure
(R = $-C(O)-C_{12}H_{25}$),
Tetradecansäure (Myristinsäure)
(R = $-C(O)-C_{13}H_{27}$).

Besonders vorteilhaft stellt R den Octanoylrest (Caprylsäurerest) bzw. den Decanoylrest (Caprinsäurerest) dar, wird also also durch die Formeln

$$R = -C(O)-C_7H_{15})$$

bzw.

$$R = -C(O)-C_9H_{19}$$

repräsentiert.

Vorteilhaft können die Emulgatoren des Typs A auch aus der Gruppe der Di- und Triglycerin-monocarbonsäuremonoester gewählt werden. Erfindungsgemäß liegen die Di- bzw. Triglycerineinheiten der erfindungsgemäßen Diglycerin-monocarbonsäuremonoester bzw. Triglycerin-monocarbonsäure-monoester als lineare, unverzweigte Moleküle, also über die jeweiligen OH-Gruppen in 1- bzw. 3-Stellung veretherte "Monoglycerinmoleküle" vor.

Ein geringer Anteil zyklischer Di- bzw. Triglycerineinheiten sowie über die OH-Gruppen in 2-Stellung veretherte Glycerinmoleküle kann geduldet werden. Es ist jedoch von Vorteil, solche Verunreinigungen so gering wie nur möglich zu halten.

Die erfindungsgemäßen Monocarbonsäuremonoester sind bevorzugt durch folgende Struktur gekennzeichnet (Substitutionspositionen angegeben):

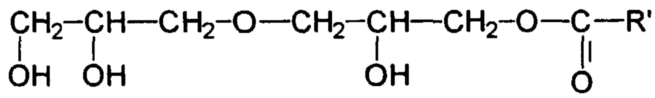

wobei R' einen Kohlenwasserstoffrest, vorteilhaft einen verzweigten oder unverzweigten Alkyl- oder Alkenylrest von 5 bis 17 C-Atomen darstellt.

Die erfindungsgemäßen Monocarbonsäureester des Triglycerins sind bevorzugt durch folgende Struktur gekennzeichnet (Substitutionspositionen angegeben):

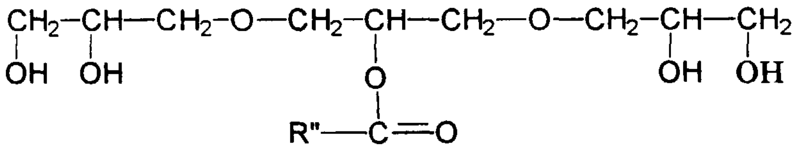

wobei R" einen Kohlenwasserstoffrest, vorteilhaft einen verzweigten oder unverzweigten Alkyl- oder Alkenylrest Alkylrest von 5 bis 17 C-Atomen darstellt.

Die diesen Estern zugrundeliegenden Säuren sind die

Hexansäure (Capronsäure)
(R' bzw. R"= $-C_5H_{11}$),
Heptansäure (Önanthsäure)
(R' bzw. R"= $-C_6H_{13}$),
Octansäure (Caprylsäure)
(R' bzw. R"= $-C_7H_{15}$),
Nonansäure (Pelargonsäure)
(R' bzw. R"= $-C_8H_{17}$),
Decansäure (Caprinsäure)
(R' bzw. R"= $-C_9H_{19}$),
Undecansäure
(R' bzw. R"= $-C_{10}H_{21}$),
10-Undecensäure (Undecylensäure)
(R' bzw. R"= $-C_{10}H_{19}$),
Dodecansäure (Laurinsäure)
(R' bzw. R"= $-C_{11}H_{23}$),
Tridecansäure
(R' bzw. R"= $-C_{12}H_{25}$),
Tetradecansäure (Myristinsäure)
(R' bzw. R"= $-C_{13}H_{27}$),
Pentadecansäure
(R' bzw. R"= $-C_{14}$H29),
Hexadecansäure (Palmitinsäure)
(R' bzw. R"= $-C_{15}H_{31}$),
Heptadecansäure (Margarinsäure)
(R' bzw. R"= $-C_{16}H_{33}$),
Octadecansäure (Stearinsäure)
(R' bzw. R"= $-C_{17}H_{35}$).

Besonders günstig werden R' und R" gewählt aus der Gruppe der unverzweigten Alkylreste mit ungeraden C-Zahlen, insbesondere mit 9, 11 und 13 C-Atomen.

Im allgemeinen sind die Monocarbonsäure-monoester des Diglycerins denen des Triglycerins bevorzugt.

Erfindungsgemäß ganz besonders günstig sind

Diglycerinmonocaprinat (DMC)
R' = 9
Triglycerinmonolaurat (TML)

R'' = 11
Diglycerinmonolaurat (DML)
R' = 11
Triglycerinmonomyristat (TMM)
R'' = 13.

Als bevorzugter erfindungsgemäßer Monocarbonsäuremonoester des Diglycerins hat sich das Diglycerinmonocaprinat (DMC) erwiesen.

Nach einer vorteilhaften Ausführungsform der vorliegenden Erfindung wird ein zusätzlicher Anteil an in anderen Stellen verestertem Di- oder Triglycerin, ebenso wie gegebenenfalls ein Anteil an den verschiedenen Diestern des Di- bzw. Triglycerins verwendet.

Vorteilhaft sind auch Triglyceryldiisostearat (Nomenklatur analog CTFA: Polyglyceryl-3-diisostearat), Isostearyldiglycerylsuccinat, Diglycerylsesquiisostearat (Nomenklatur analog CTFA: Polyglyceryl-2-sesquiisostearat), Triglycerylpolyhydroxystearat (Nomenklatur analog CTFA: Polyglyceryl-2-polyhydroxystearat).

Auch Cetearylisononanoat, Dicocoyl-pentaerythrityl-distearylcitrat, ferner die Methiconcopolyole, Cyclomethiconcopolyle, Alkylmethiconcopolyole, insbesondere Laurylmethiconcopolyol, Cetyldimethiconcopolyol, haben sich erfindungsgemäß als vorteilhaft erwiesen.

Ganz besonders vorteilhaft werden der oder die Emulgatoren des Typs A gewählt aus der Gruppe der verzweigten oder unverzweigten Alkylmonocarbonsäuren, Alkenylmonocarbonsäuren und Akylendicarbonsäuren mit 4 bis 30 Kohlenstoffatomen, insbesondere Stearinsäure, Ölsäure, Bernsteinsäure, Hexansäure (Capronsäure), Heptansäure (Önanthsäure), Octansäure (Caprylsäure), Nonansäure (Pelargonsäure), Decansäure (Caprinsäure), Undecansäure, Undecensäure (Undecylensäure), Dodecansäure (Laurinsäure), Tridecansäure, Tetradecansäure (Myristinsäure), Pentadecansäure, Hexadecansäure (Palmitinsäure), Heptadecansäure (Margarinsäure), Octadecansäure (Stearinsäure), Isostearinsäure, Behensäure. Es ist auch vorteilhaft, die Emulgatoren A aus der Gruppe der kosmetisch bzw. pharmzeutisch akzeptablen Salze der vorstehend genannten Carbonsäuren zu wählen, insbesondere der Alkali-, Ammonium-, Monoalkylammonium, Dialkylammonium-, Trialkylammonium- und Tetraalkylammoniumsalze.

Ebenfalls besonders vorteilhaft werden der oder die Emulagtoren A gewählt aus der Gruppe der mono-, oligo und polyethoxylierten Verbindungen, insbesondere der polyethoxylierten ein- oder mehrbasigen Alkohole oder Fettsäuren, beispielsweise Ceteareth-20, PEG-20-Glycerylstearat, Steareth-20, PEG-20-Stearat, PEG-30-Stearat, PEG-40-Rizinusöl, PEG-1-Glycerin-Sorbitan-Oleostearat, PEG-7 hydriertes Rizinusöl, PEG-40-Sorbitanperoleat, PEG-45-Dodecyl-Glycol-Copolymer.

Die erfindungsgemäßen Emulsionen sind vorteilhaft dadurch gekennzeichnet, daß der Emulgator A oder die Emulgatoren A in Konzentrationen von 0,01 - 20 Gew.-%, bevorzugt 0,05 - 10 Gew.-%, besonders bevorzugt 0,1 - 5 Gew.-%, jeweils bezogen auf das Gesamtgewicht der Zusammensetzung, vorliegt oder vorliegen.

Stellen die erfindungsgemäßen O/W/O-Emulsionen oder O/W-Mikroemulsionen Grundlagen für kosmetische Desodorantien/Antitranspirantien dar, so können alle gängigen Wirkstoffe vorteilhaft genutzt werden, beispielsweise Geruchsüberdecker wie die gängigen Parfümbestandteile, Geruchsabsorber, beispielsweise die in der Patentoffenlegungsschrift DE-P 40 09 347 beschriebenen Schichtsilikate, von diesen insbesondere Montmorillonit, Kaolinit, Ilit, Beidellit, Nontronit, Saponit, Hectorit, Bentonit, Smectit, ferner beispielsweise Zinksalze der Ricinolsäure. Keimhemmende Mittel sind ebenfalls geeignet, in die erfindungsgemäßen Mikroemulsionen eingearbeitet zu werden. Vorteilhafte Substanzen sind zum Beispiel 2,4,4'-Trichlor-2'-hydroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatriën-1-ol) sowie die in DE-OS 37 40 186, DE-OS 39 38 140, DE-OS 42 04 321, DE-OS 42 29 707, DE-OS 42 29 737, DE-OS 42 37 081, DE-OS 43 09 372, DE-OS 43 24 219 beschriebenen wirksamen Agenzien.

Die üblichen Antitranspiranswirkstoffe können ebenfalls vorteilhaft in den erfindungsgemäßen Zubereitungen verwendet werden, insbesondere Adstringentien, beispielsweise basische Aluminiumchloride.

Die erfindungsgemäßen kosmetischen Desodorantien können in Form von Aerosolen, also aus Aerosolbehältern, Quetschflaschen oder durch eine Pumpvorrichtung versprühbaren Präparaten vorliegen oder in Form von mittels Roll-on-Vorrichtungen auftragbaren flüssigen Zusammensetzungen, jedoch auch in Form von aus normalen Flaschen und Behältern auftragbaren Zubereitungen.

Als Treibmittel für erfindungsgemäße, aus Aerosolbehältern versprühbare kosmetische Zubereitungen, beispielsweise Desodorantien, sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorchlorkohlenwasserstoffe (FCKW).

Um niedrigviskose, insbesondere versprühbare erfindungsgemäße Mikroemulsionen zu erhalten, ist besonders

vorteilhaft, wenn die Ölphase möglichst wenig, im Idealfalle keine, Bestandteile enthält, die einen Schmelzpunkt unter 40° C haben.

Die erfindungsgemäßen kosmetischen Zubereitungen können kosmetische Hilfsstoffe enthalten, wie sie üblicherweise in solchen Zubereitungen verwendet werden, z.B. Konservierungsmittel, Bakterizide, desodorierend wirkende Substanzen, Antitranspirantien, Insektenrepellentien, Vitamine, Mittel zum Verhindern des Schäumens, Farbstoffe, Pigmente mit färbender Wirkung, Verdickungsmittel, weichmachende Substanzen, anfeuchtende und/oder feuchhaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Erfindungsgemäß enthalten die erfindungsgemäßen Zubereitungen vorteilhaft ein oder mehrere Antioxidantien. Als günstige, aber dennoch fakultativ zu verwendende Antioxidantien alle für kosmetische und/oder dermatologische Anwendungen geeigneten oder gebräuchlichen natürlichen, synthetischen und/oder partialsynthetischen Antioxiantien verwendet werden.

Besonders vorteilhaft werden die Antioxidantien gewählt aus der Gruppe bestehend aus

Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. $\alpha$-Carotin, $\beta$-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, $\gamma$-Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Pentat-, Hexa-, Heptahioninsulfoximin) in sehr geringen verträglichen Dosierungen (z.B. pmol bis $\mu$mol/kg), ferner (Metall)-Chelatoren (z.B. $\alpha$ - Hydroxyfettsäuren, Palmitinsäure, Phytinsäure, Lactoferrin), $\alpha$ - Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. $\gamma$ - Linolensäure, Linolsäure, Ölsäure), Folsäure und deren Derivate, Ubichinon und Ubichinol deren Derivate, Vitamin C und Derivate (z.B. Ascorbylpalmitate, Mg - Ascorbylphosphate, Ascorbylacetate), Tocopherole und Derivate (z.B. Vitamin-E-acetat), Vitamin A und Derivate (z.B. Vitamin A - palmitat) sowie Konyferylbenzoat des Benzoeharzes, Flavonen oder Flavonoiden, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Es ist ferner von Vorteil, den erfindungsgemäßen Zubereitungen, Enzyme, Coenzyme, insbesondere Biotin bzw. Biotinester, aber auch andere in der Kosmetik und Dermatologie übliche Substanzen dieser oder verwandter Art, beispielsweise Aktivatoren wie Citronensäure, zuzufügen.

Es ist erfindungsgemäß vorteilhaft, in den erfindungsgemäßen Zubereitungen zusätzliche öllösliche UVA-Filter und/oder UVB-Filter in der Lipidphase und/oder wasserlösliche UVA-Filter und/oder UVB-Filter in der wäßrigen Phase einzusetzen.

Vorteilhaft können die erfindungsgemäßen Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitung, um kosmetische Zubereitungen zur Verfügung zu stellen, die die Haut vor dem gesamten Bereich der ultravioletten Strahlung schützen. Sie können auch als Sonnenschutmittel dienen.

Erfindungsgemäße Zubereitungen z.B. in Form einer Sonnenschutzcreme, einer Sonnenschutzlotion oder einer Sonnenschutzmilch sind vorteilhaft und enthalten z.B. die genannten Fette, Öle, Wachse und anderen Fettkörper sowie Wasser.

Die UVB-Filter können öllöslich oder wasserlöslich sein. Vorteilhafte öllösliche UVB-Filtersubstanzen sind z.B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester,
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;
- 2,4,6-Trianilino-(p-carbo-2'-ethyl-1'-hexyloxy)-1,3,5-triazin.

Vorteilhafte wasserlösliche UVB-Filtersubstanzen sind z.B.:

- Salze der 2-Phenylbenzimidazol-5-sulfonsäure wie ihr Natrium-, Kalium - oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und deren Salze;
- Sulfonsäure-Derivate des 3-Benzylidencamphers, wie z.B. 4-(2-Oxo-3-bornylidenmethyl)benzolsulfonsäure,2-Methyl-5-(2-oxo-3-bornylidenmethyl)sulfonsäure und deren Salze.

Die Liste der genannten UVB-Filter, die in Kombination mit den erfindungsgemäßen Wirkstoffkombinationen verwendet werden können, soll selbstverständlich nicht limitierend sein.

Es kann auch von Vorteil sein, die erfindungsgemäßen Kombinationen mit UVA-Filtern zu kombinieren, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch diese Kombinationen bzw. Zubereitungen, die diese Kombinationen enthalten, sind Gegenstand der Erfindung. Es können die für die UVB-Kombination verwendeten Mengen eingesetzt werden.

Ferner ist vorteilhaft, die erfindungsgemäßen Wirkstoffkombinationen mit weiteren UVA- und/oder UVB-Filtern zu kombinieren.

Die Gesamtmenge der UVA-Filtersubstanzen kann vorteilhaft 0,1 Gew.-% bis 30 Gew.-%, vorzugsweise 0,5 bis 10 Gew.-%, insbesondere 1 bis 6 Gew.-% betragen, bezogen auf das Gesamtgewicht der Zubereitung, um kosmetische Zubereitungen zur Verfügung zu stellen, die die Haut vor dem gesamten Bereich der ultravioletten Strahlung schützen, und in denen das Stinging der $\alpha$-Hydroxycarbonsäuren bzw. $\alpha$-Ketocarbonsäuren verhindert oder drastisch vermindert ist.

Vorteilhaft sind auch solche kosmetische und dermatologische Zubereitungen, die in Form eines Sonnenschutzmittels, eines Pré-Soleil- oder Après-Soleil-Produktes vorliegen. Vorteilhaft enthalten diese zusätzlich mindestens einen UVA-Filter und/oder mindestens einen UVB-Filter.

Ferner sind auch solche kosmetische und dermatologische Zubereitungen besonders vorteilhaft, die in der Form eines Sonnenschutzmittels, eines Pré-Soleil- oder Après-Soleil-Produktes vorliegen und zusätzlich zu dem oder den UVA-Filtern und/oder dem oder den UVB-Filtern ein oder mehrere Antioxidantien enthalten.

Erfindungsgemäße kosmetische und dermatologische Zubereitungen enthalten bevorzugt anorganische Pigmente auf Basis von Metalloxiden und/oder anderen in Wasser schwerlöslichen oder unlöslichen Metallverbindungen, insbesondere der Oxide des Titans ($TiO_2$), Zinks ($ZnO$), Eisens (z.B. $Fe_2O_3$), Zirkoniums ($ZrO_2$), Siliciums ($SiO_2$), Mangans (z.B. $MnO$), Aluminiums ($Al_2O_3$), Cers (z.B. $Ce_2O_3$), Mischoxiden der entsprechenden Metalle sowie Abmischungen aus solchen Oxiden. Besonders bevorzugt handelt es sich um Pigmente auf der Basis von $TiO_2$.

Voraussetzung für die Verwendbarkeit anorganischer Pigmente für die erfindungsgemäßen Zwecke ist natürlich die kosmetische bzw. dermatologische Unbedenklichkeit der zugrundeliegenden Substanzen.

Vorteilhaft ist, den Partikeldurchmesser der verwendeten Pigmente kleiner als 100 nm zu wählen.

Erfindungsgemäß liegen die anorganischen Pigmente in hydrophober Form vor, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Reaktion gemäß

$$n\ TiO_2 + m\ (RO)_3Si\text{-}R' \rightarrow n\ TiO_2\ (\text{oberfl.})$$

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte $TiO_2$-Pigmente sind beispielsweise unter den Handelsbezeichnungen T 805 (DEGUSSA) oder M 262 (KEMIRA) oder M 160 (KEMIRA) oder MT 100 T (TAYCA) erhältlich.

Vorteilhafte $SiO_2$-Pigmente können aus der Reihe der unter den Handelsbezeichnungen AEROSIL (DEGUSSA) vertriebenen hydrophoben Pigmente, z.B. AEROSIL R 812 oder AEROSIL R 972, gewählt werden.

Erfindungsgemäße Zubereitungen sind vorteilhaft durch einen Gehalt von 0,1 bis 10 Gew.-%, insbesondere 0,5 - 5,0 Gew.-%, an hydrophoben anorganischen Pigmenten gekennzeichnet, jeweils bezogen auf das Gesamtgewicht der Zusammensetzung.

Die erfindungsgemäßen Emulsionen können grundsätzlich alle kosmetischen Verwendungszwecke erfüllen, die üblicherweise Emulsionen zu erfüllen haben, beispielsweise Tagesrèmes, Nachtcrèmes, Hand- oder Körpercrèmes, Sonnenschutzformulierungen, Nährstoffcrèmes, Liposomencrèmes, Vitamincrèmes usw.

Es dabei sowohl vorteilhaft, die erfindungsgemäßen Zubereitungen auf dem Gebiete der pflegenden Kosmetik als auch auf dem Gebiete der dekorativen Kosmetik zu verwenden.

Es ist aber gegebenenfalls auch vorteilhaft, erfindungsgemäße Zubereitungen als Trägersubstanz dermatologischer oder topischer Zubereitungen zu verwenden.

Nachfolgend soll kurz noch auf einige Besonderheiten und Unterschiede der Voraussetzungen für erfindungsgemäß O/W-Emulsionen, O/W-Mikroemulsionen bzw O/W/O-Emulsionen eingegangen werden.

Öle und Fette unterscheiden sich unter anderem in ihrer Polarität, welche schwierig zu definieren ist. Es wurde bereits vorgeschlagen, die Grenzflächenspannung gegenüber Wasser als Maß für den Polaritätsindex eines Öls bzw. einer Ölphase anzunehmen. Dabei gilt, daß die Polarität der betreffenden Ölphase umso größer ist, je niedriger die Grenzflächenspannung zwischen dieser Ölphase und Wasser ist. Erfindungsgemäß wird die Grenzflächenspannung als ein mögliches Maß für die Polarität einer gegebenen Ölkomponente angesehen.

Die Grenzflächenspannung ist diejenige Kraft, die an einer gedachten, in der Grenzfläche zwischen zwei Phasen befindlichen Linie der Länge von einem Meter wirkt. Die physikalische Einheit für diese Grenzflächenspannung errechnet sich klassisch nach der Beziehung Kraft/Länge und wird gewöhnlich in mN/m (Millinewton geteilt durch Meter) wiedergegeben. Sie hat positives Vorzeichen, wenn sie das Bestreben hat, die Grenzfläche zu verkleinern. Im umgekehrten Falle hat sie negatives Vorzeichen.

Als Grenze, unterhalb derer eine Ölphase als "polar" und oberhalb derer eine Ölphase als "unpolar" gilt, werden erfindungsgemäß 30 mN/m angesehen.

Erfindungsgemäß wird für O/W-Mikroemulsionen die Ölphase vorteilhaft gewählt aus der Gruppe der polaren Ölkomponenten, welche eine Polarität zwischen 10 und 30 mN/m aufweisen, wobei gewährleistet sein muß, daß mindestens eine nichtpolare Ölkomponente vorhanden ist.

Vorteilhafte O/W-Mikroemulsionen werden erhalten, wenn die Ölphase gewählt wird aus der Gruppe der polaren Ölkomponenten, besonders bevorzugt aus der Gruppe der natürlichen, synthetischen oder halbsynthetischen Ölkomponenten, welche eine Polarität zwischen 10 und 20 mN/m aufweisen, wobei gewährleistet sein muß, daß mindestens eine nichtpolare Ölkomponente vorhanden ist.

Vorteilhaft ist auch, polare pflanzliche Öle als polare Öle der erfindungsgemäßen O/W-Emulsionen zu verwenden. Die pflanzlichen Öle können vorteilhaft gewählt werden aus der Gruppe der Öle der Pflanzenfamilien Euphorbiaceae, Poaceae, Fabaceae, Brassicaceae, Pedalaceae, Asteraceae, Linaceae, Flacourticaceae, Violales, vorzugsweise gewählt aus der Gruppe natives Rizinusöl, Weizenkeimöl, Traubenkernöl, Kukuinußöl, Safloröl, Distelöl, Nachtkerzenöl und weiteren Ölen, welche mindestens 1,5 Gew.-% an Linolsäureglyceriden enthalten.

Im Gegenzuge sollen erfindungsgemäße O/W/O-Emulsionen von solchen Ölkomponenten nur untergeordnete Mengen aufweisen und statt deren hauptsächlich solche, deren Polaritätswert höher liegt als 30 mN/m. Als vorteilhaft haben sich gleichermaßen natürliche, synthetische und halbsynthetische Öle, Fette und Wachse erwiesen.

Der Zusatz von Elektrolyten bewirkt eine Veränderung des Löslichkeitsverhaltens eines hydrophilen Emulgators. Die hydrophilen Emulgatoren mit den vorab beschriebenen Strukturen bzw. Eigenschaften durchlaufen eine partielle Phaseninversion, in der es zu einer Solubilisierung von Wasser durch die Ölphase kommt, welche in einer stabilen Mikroemulsion oder, im gewünschten Falle, auch einer stabilen O/W/O-Emulsion resultiert.

Die erfindungsgemäßen Mikroemulsionen enthalten daher vorteilhaft Elektrolyte, insbesondere eines oder mehrere Salze mit folgenden Anionen: Chloride, ferner anorganische Oxo-Element-Anionen, von diesen insbesondere Sulfate, Carbonate, Phosphate, Borate und Aluminate. Auch auf organischen Anionen basierende Elektrolyte können vorteilhaft verwendet werden, beispielsweise Lactate, Acetate, Benzoate, Propionate, Tartrate, Citrate und andere mehr. Vergleichbare Effekte sind auch durch Ethylendiamintetraessigsäure und deren Salze zu erzielen.

Als Kationen der Salze werden bevorzugt Ammonium,- Alkylammonium,- Alkalimetall-, Erdalkalimetall,- Magnesium-, Eisen- bzw. Zinkionen verwendet. Es bedarf an sich keiner Erwähnung, daß in Kosmetika nur physiologisch unbedenkliche Elektrolyte verwendet werden sollten. Spezielle medizinische Anwendungen der erfindungsgemäßen Mikroemulsionen können andererseits, wenigstens grundsätzlich, die Verwendung von Elektrolyten bedingen, welche nicht ohne ärztliche Aufsicht verwendet werden sollten.

Besonders bevorzugt sind Kaliumchlorid, Kochsalz, Magnesiumsulfat, Zinksulfat und Mischungen daraus. Ebenfalls vorteilhaft sind Salzmischungen wie sie im natürlichen Salz vom Toten Meer auftreten.

Die Konzentration des Elektrolyten oder der Elektrolyte sollte etwa 0,01 - 10,0 Gew.-%, besonders vorteilhaft etwa 0,03 - 8,0 Gew.% betragen, bezogen auf das Gesamtgewicht der Zubereitung.

Die Emulgatoren des Typs A können landsläufig als O/W-Emulgatoren angesehen werden. Ein Gehalt von etwa 5 - 10 Gew.-% an üblichen W/O-Emulgatoren fördert in vorteilhafter Weise die Bildung von O/W/O-Emulsionen, ein Gehalt von deutlich über 10 Gew.-% an solchen Emulgatoren führt zur Destabilisierung der O/W/O-Emulsionen.

Ferner ist gewünschtenfalls für die Herstellung erfindungsgemäßer O/W/O-Emulsionen von Vorteil, hydrophile und/oder lipophile Gelbildner einzusetzen. Diese tragen zwar in der Regel nicht zur Bildung multipler Tröpfchen bei, fördern aber die Stabilität einmal gebildeter multipler Tröpfchen.

Wenn bei einem erfindungsgemäßen Herstellungsverfahren für O/W/O-Emulsionen der pH-Wert variiert werden soll, um ein ansonsten vorbestimmtes System in den Phaseninversionsbereich zu bringen, so ist von Vorteil, zu Beginn des Verfahrens zunächst möglichst geringe Elektrolytkonzentration in der Wasserphase einzusetzen, möglichst zunächst ganz darauf zu verzichten. Weiterhin ist von Vorteil, den Emulgator A in der Ölphase vorzulegen, beispielsweise für Stearinsäure im Konzentrationsbereich von 0,5 - 5 Gew.-%, insbesondere 2 Gew.-%. Vorteilhaft ist die Gegenwart eines nicht unter die Definition des Emulgators A fallenden Emulgators im Konzentrationsbereich von ca. 5 - 10 Gew.-%, insbesondere etwa 7 Gew.-%.

Die Variation des pH-Wertes sollte vorteilhaft erst erfolgen, wenn die W/O-Emulsion sich gebildet hat, beispielsweise durch Zufügen von NaOH.

Dabei liegt im allgemeinen Fachwissen des Fachmannes und bedarf keinerlei erfinderischen Dazutuns, für einen gegebenen Emulgator oder ein gegebenes Emulgatorsystem in einem gegebenen Wasser/Ölphasensystem den Temperatur- bzw. pH-Bereich zu ermitteln, in welchem Phaseninversion stattfindet. Als allgemeiner Richtwert für den PIT bei üblichen Emulgatorkonzentrationen kann ein Temperaturbereich von etwa 40 - 90° C angegeben werden. Im allgemeinen sinkt der PIT bei steigender Emulgatorkonzentration.

Es können während dieses Verfahrens gewünschtenfalls ferner die in der Kosmetik oder medizinischen Galenik üblichen Grund-, Hilfs-, Zusatz-, und/oder Wirkstoffe zugegeben werden. Es ist dem Fachmann klar, zu welchem Zeitpunkt solche Stoffe dem Prozeß beigegeben werden können, ohne daß die Eigenschaften der zu erzielenden Emulsion wesentlich beeinträchtigt werden.

Die nachfolgenden Beispiele sollen das Wesen der vorliegenden Erfindung näher umreißen, ohne die Erfindung einzuschränken.

**Beispiel 1**

O/W-Mikroemulsion

| | Gew.-% |
|---|---|
| Ceteareth-12 | 8,00 |
| Cetearylisononanoat | 20,00 |
| Cetearylalkohol | 4,00 |
| Uvinul® T150 | 2,00 |
| Parsol® 1789 | 1,00 |
| Eusolex® 232 | 4,80 |
| | pH=7,5 |
| Farbstoffe, Parfum, Konservierungsmittel | q.s. |
| Wasser | ad 100,00 |

Die UV-Filter werden in der der Ölphase gelöst und mit den übrigen Bestandteilen der Ölphase vereinigt, worauf das Gemisch homogenisiert, dann mit der Wasserphase vereinigt und auf eine Temperatur von 80 - 85° C (d.h., in den Phaseninversionstemperaturbereich des Systems) gebracht, hernach auf Raumtemperatur abgekühlt (also aus dem Phaseninversionstemperaturbereich des Systems wieder heraus gebracht).

**Beispiel 2**

O/W-Mikroemulsion

| | Gew.-% |
|---|---|
| Ceteareth-12 | 12,00 |
| Cetearylisononanoat | 20,00 |
| Cetearylalkohol | 6,00 |
| Uvinul® T150 | 2,00 |
| Eusolex® 6300 | 3,00 |
| Eusolex® 232 | 4,80 |
| | pH=7,5 |
| Farbstoffe, Parfum, Konservierungsmittel | q.s. |
| Wasser | ad 100,00 |

Die UV-Filter werden in der der Ölphase gelöst und mit den übrigen Bestandteilen der Ölphase vereinigt, worauf das Gemisch homogenisiert, dann mit der Wasserphase vereinigt und auf eine Temperatur von 80 - 85° C (d.h., in den Phaseninversionstemperaturbereich des Systems) gebracht, hernach auf Raumtemperatur abgekühlt (also aus dem Phaseninversionstemperaturbereich des Systems wieder heraus gebracht).

**Beispiel 3**

O/W-Mikroemulsion

| | Gew.-% |
|---|---|
| Ceteareth-12 | 8,00 |
| Cetearylisononanoat | 20,00 |
| Cetearylalkohol | 4,00 |
| Uvinul® T150 | 4,80 |
| $MgSO_4$ | 3,00 |
| Farbstoffe, Parfum, Konservierungsmittel | q.s. |
| Wasser | ad 100,00 |

Die UV-Filter werden in der der Ölphase gelöst und mit den übrigen Bestandteilen der Ölphase vereinigt, worauf das Gemisch homogenisiert, dann mit der Wasserphase vereinigt und auf eine Temperatur von 80 - 85° C (d.h., in den Phaseninversionstemperaturbereich des Systems) gebracht, hernach auf Raumtemperatur abgekühlt (also aus dem Phaseninversionstemperaturbereich des Systems wieder heraus gebracht).

**Beispiel 4**

O/W-Mikroemulsion

| | Gew.-% |
|---|---|
| Ceteareth-12 | 12,00 |
| Cetearylisononanoat | 20,00 |
| Cetearylalkohol | 6,00 |
| Uvinul® T150 | 4,80 |
| Parsol® 1789 | 2,00 |
| $TiO_2$ | 2,00 |
| $MgSO_4$ | 3,00 |
| Farbstoffe, Parfum, Konservierungsmittel | q.s. |
| Wasser | ad 100,00 |

Die UV-Filter werden in der der Ölphase gelöst und mit den übrigen Bestandteilen der Ölphase vereinigt, worauf das Gemisch homogenisiert, dann mit der Wasserphase vereinigt und auf eine Temperatur von 80 - 85° C (d.h., in den Phaseninversionstemperaturbereich des Systems) gebracht, hernach auf Raumtemperatur abgekühlt (also aus dem Phaseninversionstemperaturbereich des Systems wieder heraus gebracht).

**Beispiel 5**

O/W-Mikroemulsion

| | Gew.-% |
|---|---|
| Ceteareth-12 | 8,00 |
| Cetearylisononanoat | 10,00 |
| Cetearylalkohol | 4,00 |
| Eusolex® 232 | 5,00 |
| Uvinul® T150 | 1,00 |
| Parsol® 1789 | 4,00 |
| | pH=7,5 |
| Farbstoffe, Parfum, Konservierungsmittel | q.s. |
| Wasser | ad 100,00 |

Die UV-Filter werden in der der Ölphase gelöst und mit den übrigen Bestandteilen der Ölphase vereinigt, worauf das Gemisch homogenisiert, dann mit der Wasserphase vereinigt und auf eine Temperatur von 80 - 85° C (d.h., in den Phaseninversionstemperaturbereich des Systems) gebracht, hernach auf Raumtemperatur abgekühlt (also aus dem Phaseninversionstemperaturbereich des Systems wieder heraus gebracht).

**Beispiel 6**

O/W/O-Emulsion

| | Gew.-% |
|---|---|
| Glycerylisostearat | 4,00 |
| Cetearylisononaoat | 20,00 |
| Stearinsäure | 2,00 |
| Uvinul® T150 | 2,00 |
| Parsol® 1789 | 3,00 |
| NaOH | ad pH 7,0 |
| Farbstoffe, Parfum, Konservierungsmittel | q.s. |
| Wasser | ad 100,00 |

Die UV-Filter werden in der Ölphase gelöst und mit der restlichen Ölphase vereinigt. Wasser wird zugegeben und das System auf ca. 40° C erwärmt. NaOH wird zugegeben bis ein pH-Wert von 7 erreicht ist, hernach wird auf Raumtemperatur abgekühlt.

**Beispiel 7**

O/W-Mikroemulsion

| | Gew.-% |
|---|---|
| Ceteareth-12 | 8,00 |
| Cetearylisononanoat | 10,00 |
| Mineralöl | 10,00 |
| Cetearylalkohol | 4,00 |
| Uvinul® T150 | 2,00 |
| Parsol® 1789 | 1,00 |
| Eusolex® 6300 | 3,00 |
| $TiO_2$ | 4,80 |
| $MgSO_4$ | 3,00 |
| Farbstoffe, Parfum, Konservierungsmittel | q.s. |
| Wasser | ad 100,00 |

Das anorganische Mikropigment sowie das Uvinul® T150 werden im Mineralöl vorsuspendiert und mit der restlichen Ölphase vereinigt, worauf diese homogenisiert und dann mit der Wasserphase vereinigt und auf eine Temperatur von 80 - 85° C (d.h., in den Phaseninversionstemperaturbereich des Systems) gebracht, hernach auf Raumtemperatur abgekühlt (also aus dem Phaseninversionstemperaturbereich des Systems wieder heraus gebracht) wird.

**Beispiel 8**

O/W-Mikroemulsion

| | Gew.-% |
|---|---|
| Ceteareth-12 | 12,00 |
| Cetearylisononanoat | 10,00 |
| Mineralöl | 10,00 |
| Cetearylalkohol | 6,00 |
| Uvinul® T150 | 2,00 |
| Parsol® 1789 | 1,00 |
| $TiO_2$ | 4,80 |
| $MgSO_4$ | 3,00 |
| Farbstoffe, Parfum, Konservierungsmittel | q.s. |
| Wasser | ad 100,00 |

Das anorganische Mikropigment und das Uvinul® T150 werden im Mineralöl vorsuspendiert und mit der restlichen Ölphase vereinigt, worauf diese homogenisiert und dann mit der Wasserphase vereinigt und auf eine Temperatur von 80 - 85° C (d.h., in den Phaseninversionstemperaturbereich des Systems) gebracht, hernach auf Raumtemperatur abgekühlt (also aus dem Phaseninversionstemperaturbereich des Systems wieder heraus gebracht) wird.

**Beispiel 9**

O/W-Mikroemulsion

| | Gew.-% |
|---|---|
| Ceteareth-12 | 8,00 |
| Cetearylisononanoat | 10,00 |
| Mineralöl | 10,00 |
| Cetearylalkohol | 4,00 |
| $TiO_2$ | 5,00 |
| $MgSO_4$ | 3,00 |
| Farbstoffe, Parfum, Konservierungsmittel | q.s. |
| Wasser | ad 100,00 |

Das anorganische Mikropigment wird im Mineralöl vorsuspendiert und mit der restlichen Ölphase vereinigt, worauf diese homogenisiert und dann mit der Wasserphase vereinigt und auf eine Temperatur von 80 - 85° C (d.h., in den Phaseninversionstemperaturbereich des Systems) gebracht, hernach auf Raumtemperatur abgekühlt (also aus dem Phaseninversionstemperaturbereich des Systems wieder heraus gebracht) wird.

**Beispiel 10**

O/W/O-Emulsion

| | Gew.-% |
|---|---|
| Glycerylisostearat | 5,00 |
| Mineralöl | 25,00 |
| Stearinsäure | 2,00 |
| $TiO_2$ | 2,00 |
| NaOH | ad pH 7,0 |
| Farbstoffe, Parfum, Konservierungsmittel | q.s. |
| Wasser | ad 100,00 |

Das anorganische Mikropigment wird in der Ölphase dispergiert. Wasser wird zugegeben und das System auf ca. 40° C erwärmt. NaOH wird zugegeben bis ein pH-Wert von 7 erreicht ist, hernach wird auf Raumtemperatur abgekühlt.

**Beispiel 11**

O/W/O-Emulsion

| | Gew.-% |
|---|---|
| Polyglyceryl-2-polyhydroxystearat | 7,00 |
| Cetearylisononaoat | 12,50 |
| Mineralöl | 12,50 |
| Stearinsäure | 2,00 |
| $TiO_2$ | 2,00 |
| NaOH | ad pH 7,0 |
| Farbstoffe, Parfum, Konservierungsmittel | q.s. |
| Wasser | ad 100,00 |

Das anorganische Mikropigment wird im Mineralöl vorsuspendiert und mit der restlichen Ölphase vereinigt. Wasser wird zugegeben und das System auf ca. 40° C erwärmt. NaOH wird zugegeben bis ein pH-Wert von 7 erreicht ist, hernach wird auf Raumtemperatur abgekühlt.

**Beispiel 12**

O/W/O-Emulsion

| | Gew.-% |
|---|---|
| Polyglyceryl-2-polyhydroxystearat | 5,00 |
| Cetearylisononaoat | 12,50 |
| Mineralöl | 12,50 |
| Stearinsäure | 2,00 |
| $TiO_2$ | 2,00 |
| NaOH | ad pH 7,0 |
| Farbstoffe, Parfum, Konservierungsmittel | q.s. |
| Wasser | ad 100,00 |

Das anorganische Mikropigment wird in der Ölphase dispergiert. Wasser wird zugegeben und das System auf ca. 40° C erwärmt. NaOH wird zugegeben bis ein pH-Wert von 7 erreicht ist, hernach wird auf Raumtemperatur abgekühlt.

**Beispiel 13**

O/W-Makroemulsion

| | Gew.-% |
|---|---|
| Polyglyceryl-2-polyhydroxystearat | 2,00 |
| Cetearylisononaoat | 12,50 |
| Mineralöl | 12,50 |
| Stearinsäure | 2,00 |
| $TiO_2$ | 2,00 |
| NaOH | ad pH 7,0 |
| Farbstoffe, Parfum, Konservierungsmittel | q.s. |
| Wasser | ad 100,00 |

Das anorganische Mikropigment wird im Mineralöl vorsuspendiert und mit der restlichen Ölphase vereinigt. Wasser wird zugegeben und das System auf ca. 40° C erwärmt. NaOH wird zugegeben bis ein pH-Wert von 7 erreicht ist, hernach wird auf Raumtemperatur abgekühlt.

**Beispiel 14**

O/W-Mikroemulsion

| | Gew.-% |
|---|---|
| Ceteareth-12 | 8,00 |
| Cetearylisononanoat | 20,00 |
| Cetearylalkohol | 4,00 |
| Eusolex® 232 | 4,80 |
| $MgSO_4$ | 3,00 |
| | pH=5,0 |
| Farbstoffe, Parfum, Konservierungsmittel | q.s. |
| Wasser | ad 100,00 |

Die Bestandteile der Ölphase werden vereinigt und homogenisiert, dann mit der Wasserphase vereinigt und auf eine Temperatur von 80 - 85° C (d.h., in den Phaseninversionstemperaturbereich des Systems) gebracht, hernach auf Raumtemperatur abgekühlt (also aus dem Phaseninversionstemperaturbereich des Systems wieder heraus gebracht).

**Beispiel 15**

O/W-Mikroemulsion

| | Gew.-% |
|---|---|
| Ceteareth-12 | 12,00 |
| Cetearylisononanoat | 20,00 |
| Cetearylalkohol | 6,00 |
| Eusolex® 232 | 4,80 |
| Parsol ® 1789 | 2,00 |
| $MgSO_4$ | 3,00 |
| | pH=5,0 |
| Farbstoffe, Parfum, Konservierungsmittel | q.s. |
| Wasser | ad 100,00 |

Die Bestandteile der Ölphase werden vereinigt und homogenisiert, dann mit der Wasserphase vereinigt und auf eine Temperatur von 80 - 85° C (d.h., in den Phaseninversionstemperaturbereich des Systems) gebracht, hernach auf Raumtemperatur abgekühlt (also aus dem Phaseninversionstemperaturbereich des Systems wieder heraus gebracht).

**Beispiel 16**

O/W-Mikroemulsion

| | Gew.-% |
|---|---|
| Ceteareth-12 | 8,00 |
| Cetearylisononanoat | 10,00 |
| Mineralöl | 10,00 |
| Cetearylalkohol | 4,00 |
| Uvinul® T150 | 4,80 |
| $MgSO_4$ | 3,00 |
| Farbstoffe, Parfum, Konservierungsmittel | q.s. |
| Wasser | ad 100,00 |

Das Uvinul® T150 wird im Mineralöl vorsuspendiert und mit der restlichen Ölphase vereinigt, worauf diese und homogenisiert und dann mit der Wasserphase vereinigt und auf eine Temperatur von 80 - 85° C (d.h., in den Phaseninversionstemperaturbereich des Systems) gebracht, hernach auf Raumtemperatur abgekühlt (also aus dem Phaseninversionstemperaturbereich des Systems wieder heraus gebracht) wird.

**Beispiel 17**

O/W-Mikroemulsion

| | Gew.-% |
|---|---|
| Ceteareth-12 | 12,00 |
| Cetearylisononanoat | 10,00 |
| Mineralöl | 10,00 |
| Cetearylalkohol | 6,00 |
| Eusolex ® 6300 | 2,00 |
| Parsol® 1789 | 0,50 |
| Uvinul® T150 | 4,80 |
| $MgSO_4$ | 3,00 |
| Farbstoffe, Parfum, Konservierungsmittel | q.s. |
| Wasser | ad 100,00 |

Die Uvinul® T150 wird im Mineralöl vorsuspendiert und mit der restlichen Ölphase vereinigt, worauf diese und homogenisiert und dann mit der Wasserphase vereinigt und auf eine Temperatur von 80 - 85° C (d.h., in den Phaseninversionstemperaturbereich des Systems) gebracht, hernach auf Raumtemperatur abgekühlt (also aus dem Phaseninversionstemperaturbereich des Systems wieder heraus gebracht) wird.

**Beispiel 18**

O/W-Mikroemulsion

| | Gew.-% |
|---|---|
| Ceteareth-12 | 8,00 |
| Cetearylisononanoat | 10,00 |
| Cetearylalkohol | 4,00 |
| Eusolex® 232 | 5,00 |
| Uvinul® T150 | 1,00 |
| $MgSO_4$ | 3,00 |
| | pH=5,0 |
| Farbstoffe, Parfum, Konservierungsmittel | q.s. |
| Wasser | ad 100,00 |

Die Bestandteile der Ölphase werden vereinigt und homogenisiert, dann mit der Wasserphase vereinigt und auf eine Temperatur von 80 - 85° C (d.h., in den Phaseninversionstemperaturbereich des Systems) gebracht, hernach auf Raumtemperatur abgekühlt (also aus dem Phaseninversionstemperaturbereich des Systems wieder heraus gebracht).

**Beispiel 19**

O/W-Mikroemulsion

| | Gew.-% |
|---|---|
| Ceteareth-12 | 8,00 |
| Cetearylisononanoat | 10,00 |
| Mineralöl | 10,00 |
| Cetearylalkohol | 4,00 |
| Uvinul® T150 | 5,00 |
| $MgSO_4$ | 3,00 |
| Farbstoffe, Parfum, Konservierungsmittel | q.s. |
| Wasser | ad 100,00 |

Das Uvinul® T150 wird im Mineralöl vorsuspendiert und mit der restlichen Ölphase vereinigt, worauf diese und homogenisiert und dann mit der Wasserphase vereinigt und auf eine Temperatur von 80 - 85° C (d.h., in den Phaseninversionstemperaturbereich des Systems) gebracht, hernach auf Raumtemperatur abgekühlt (also aus dem Phaseninversionstemperaturbereich des Systems wieder heraus gebracht) wird.

**Beispiel 20**

O/W/O-Emulsion

| | Gew.-% |
|---|---|
| Glycerylisostearat | 5,00 |
| Mineralöl | 25,00 |
| Stearinsäure | 2,00 |
| Uvinul® T150 | 2,00 |
| NaOH | ad pH 7,0 |
| Farbstoffe, Parfum, Konservierungsmittel | q.s. |
| Wasser | ad 100,00 |

Das Uvinul® T150 wird in der Ölphase dispergiert. Wasser wird zugegeben und das System auf ca. 40° C erwärmt. NaOH wird zugegeben bis ein pH-Wert von 7 erreicht ist, hernach wird auf Raumtemperatur abgekühlt.

**Beispiel 21**

O/W/O-Emulsion

| | Gew.-% |
|---|---|
| Polyglyceryl-2-polyhydroxystearat | 7,00 |
| Cetearylisononaoat | 12,50 |
| Mineralöl | 12,50 |
| Stearinsäure | 2,00 |
| Uvinul® T150 | 2,00 |
| Parsol® 1789 | 2,00 |
| Eusolex® 232 | 2,00 |
| NaOH | ad pH 7,0 |
| Farbstoffe, Parfum, Konservierungsmittel | q.s. |
| Wasser | ad 100,00 |

Die UV-Filtersubstanzen werden im Mineralöl vorsuspendiert und mit der restlichen Ölphase vereinigt. Wasser wird zugegeben und das System auf ca. 40° C erwärmt. NaOH wird zugegeben bis ein pH-Wert von 7 erreicht ist, hernach wird auf Raumtemperatur abgekühlt.

**Beispiel 22**

O/W/O-Emulsion

| | Gew.-% |
|---|---|
| Polyglyceryl-2-polyhydroxystearat | 5,00 |
| Cetearylisononaoat | 12,50 |
| Mineralöl | 12,50 |
| Stearinsäure | 2,00 |
| Eusolex® 232 | 2,00 |
| Uvinul® T150 | 2,00 |
| Eusolex® 6300 | 2,00 |
| Parsol® 1789 | 1,00 |
| NaOH | ad pH 7,0 |
| Farbstoffe, Parfum, Konservierungsmittel | q.s. |
| Wasser | ad 100,00 |

Die UV-Filtersubstanzen werden in der Ölphase dispergiert. Wasser wird zugegeben und das System auf ca. 40° C erwärmt. NaOH wird zugegeben bis ein pH-Wert von 7 erreicht ist, hernach wird auf Raumtemperatur abgekühlt.

**Beispiel 23**

O/W-Makroemulsion

| | Gew.-% |
|---|---|
| Polyglyceryl-2-polyhydroxystearat | 2,00 |
| Cetearylisononaoat | 12,50 |
| Mineralöl | 12,50 |
| Stearinsäure | 2,00 |
| Uvinul®T150 | 2,00 |
| NaOH | ad pH 7,0 |
| Farbstoffe, Parfum, Konservierungsmittel | q.s. |
| Wasser | ad 100,00 |

Das Uvinul®T150 wird im Mineralöl vorsuspendiert und mit der restlichen Ölphase vereinigt. Wasser wird zugegeben und das System auf ca. 40° C erwärmt. NaOH wird zugegeben bis ein pH-Wert von 7 erreicht ist, hernach wird auf Raumtemperatur abgekühlt.

**Patentansprüche**

1. O/W-Emulsionen, insbesondere O/W-Mikroemulsionen, oder O/W/O-Emulsionen oder O/W/O'-Emulsionen, enthaltend

- eine Wasserphase,
- gegebenenfalls übliche, in Wasser lösliche oder dispergierbare Substanzen,
- eine Ölphase,
- mindestens einen Emulgator (Emulgator A), gewählt aus der Gruppe der Emulgatoren mit folgenden Eigenschaften

    - ihre Lipophilie ist entweder abhängig vom pH-Wert, dergestalt daß durch Erhöhung oder Senkung des pH-Wertes die Lipophilie zunimmt oder abnimmt, wobei unerheblich ist, welche der beiden Möglichkeiten der Änderung der Lipophilie durch die Erhöhung bzw. Senkung des pH-Wertes bewirkt wird, und/oder
    - ihre Lipophilie ist abhängig von der Temperatur, dergestalt, daß die Lipophilie mit steigender Temperatur zunimmt und deren Hydrophilie mit sinkender Temperatur zunimmt,

- ferner gegebenenfalls weitere in der Ölphase lösliche oder dispergierbare Substanzen, darunter bevorzugt solche, gewählt aus der Gruppe der nicht unter die Definition des Emulgators A fallende Emulgatoren, insbesondere solche, die vorwiegend als W/O-Emulgatoren wirken.

**2.** Verfahren zur Herstellung von O/W-Emulsionen, insbesondere O/W-Mikroemulsionen, oder O/W/O-Emulsionen oder O/W/O'-Emulsionen, dadurch gekennzeichnet, daß eine Wasserphase, gegebenenfalls übliche, in Wasser lösliche oder dispergierbare Substanzen, eine Ölphase sowie

- mindestens ein Emulgator (Emulgator A), gewählt aus der Gruppe der O/W-Emulgatoren mit folgenden Eigenschaften

    - ihre Lipophilie ist abhängig vom pH-Wert, dergestalt daß durch Erhöhung oder Senkung des pH-Wertes die Lipophilie zunimmt oder abnimmt, wobei unerheblich ist, welche der beiden Möglichkeiten der Änderung der Lipophilie durch die Erhöhung bzw. Senkung des pH-Wertes bewirkt wird, und
    - ihre Lipophilie ist gegebenenfalls zusätzlich abhängig von der Temperatur, dergestalt, daß die Lipophilie mit steigender Temperatur zunimmt und deren Hydrophilie mit sinkender Temperatur zunimmt,

ferner gegebenenfalls weitere in der Ölphase lösliche oder dispergierbare Substanzen, darunter bevorzugt solche, gewählt aus der Gruppe der nicht unter die Definition des Emulgators A fallende Emulgatoren, zusammengegeben werden und unter Agitation ein Gemisch gebildet wird, dergestalt, daß

- dieses Gemisch durch geeignete Wahl der Parameter, gewählt aus der Gruppe pH-Wert, Temperatur und der Konzentration bzw. Konzentrationen mindestens eines der gewählten Emulgatoren, in den Phaseninversionsbereich gebracht wird, in welchem sich W/O-Emulsionen in O/W-Emulsionen umwandeln,
- durch Variation mindestens eines Parameters, gewählt aus der Gruppe pH-Wert, Temperatur und der Konzentration bzw. Konzentrationen mindestens eines der gewählten Emulgatoren, die gebildete W/O-Emulsion aus dem Phaseninversionsbereich heraus gebracht wird, in welchem sich eine gebildete W/O-Emulsion in eine O/W-Emulsion umwandelt, wodurch eine O/W-Emulsion oder O/W-Mikroemulsion erzeugt wird.
- gegebenenfalls durch geeignete Wahl der Rahmenbedingungen eine weitere Phaseninversion zur O/W/O-Emulsion eingeleitet wird,
- das Gemisch gegebenenfalls weiteren Aufbereitungsschritten, insbesondere einem oder mehreren Homogenisierungsschritten unterworfen wird.

**3.** Verfahren nach Anspruch 2 zur Herstellung von O/W/O-Emulsionen, dadurch gekennzeichnet, daß der Ölphasenanteil höher als etwa 15 Gew.-%, insbesondere höher als etwa 20 Gew.-%, bezogen auf das Gesamtgewicht der Zubereitung liegt, mehr als etwa 5 Gew.-%, insbesondere etwa 5 - 10 Gew.-% eines zusätzlichen, bei Raumtemperatur als W/O-Emulgator wirkenden Emulgators vorliegen und/oder wenn die Ölphase einen niedrigen Anteil an polaren Ölen aufweist oder weitgehend frei von solchen Ölen ist.

**4.** Verfahren nach Anspruch 2 zur Herstellung von O/W-Mikroemulsionen, dadurch gekennzeichnet, daß der Ölphasenanteil unter etwa 20 Gew.-%, insbesondere unter etwa 15 Gew.-%, bezogen auf das Gesamtgewicht der Zubereitung liegt, weniger als etwa 5 Gew.-% eines zusätzlichen, bei Raumtemperatur als W/O-Emulgator wirkenden Emulgators vorliegen und/oder wenn die Ölphase einen hohen Anteil an polaren Ölen aufweist.

**5.** O/W/O-Emulsionen oder O/W-Mikroemulsionen nach Anspruch 1, dadurch gekennzeichnet, daß der Emulgator A

gewählt wird aus der Gruppe der Sorbitanester und Saccharoseester, insbesondere der verzweigten und unverzweigten Alkylester und Alkenylester mit Kohlenstoffketten von 4 - 24 Kohlenstoffatomen, bevorzugt Sorbitanstearat, Sorbitanoleat, Glycerylsorbitanstearat, Saccharosemonostearat, Saccharosemonolaurat, Saccharosepalmitat, der Monoglycerin-monocarbonsäure-monoester, der Di- und Triglycerin-monocarbonsäure-monoester, ferner Triglyceryldiisostearat, Isostearyldiglycerylsuccinat, Diglycerylsesquiisostearat, Triglycerylpolyhydroxystearat, Cetearylisononanoat, Dicocoyl-pentaerythriryl-distearylcitrat, ferner die Methiconcopolyole, Cyclomethiconcopolyle, Alkylmethiconcopolyole, Laurylmethiconcopolyol, Cetyldimethiconcopolyol, sowie der Gruppe der verzweigten oder unverzweigten Alkylmonocarbonsäuren, Alkenylmonocarbonsäuren und Akylendicarbonsäuren mit 4 bis 30 Kohlenstoffatomen, insbesondere Stearinsäure, Ölsäure, Bernsteinsäure, Hexansäure (Capronsäure), Heptansäure (Önanthsäure), Octansäure (Caprylsäure), Nonansäure (Pelargonsäure), Decansäure (Caprinsäure), Undecansäure, Undecensäure (Undecylensäure), Dodecansäure (Laurinsäure), Tridecansäure, Tetradecansäure (Myristinsäure), Pentadecansäure, Hexadecansäure (Palmitinsäure), Heptadecansäure (Margarinsäure), Octadecansäure (Stearinsäure), Isostearinsäure, Behensäure bzw. deren kosmetisch bzw. pharmzeutisch akzeptablen Salze, insbesondere der Alkali-, Ammonium-, Monoalkylammonium, Dialkylammonium-, Trialkylammonium- und Tetraalkylammoniumsalze.

**6.** O/W/O-Emulsionen oder O/W-Mikroemulsionen nach Anspruch 1, dadurch gekennzeichnet, daß der Emulgator A oder die Emulgatoren A in Konzentrationen von 0,01 - 20 Gew.-%, bevorzugt 0,05 - 10 Gew.-%, besonders bevorzugt 0,1 - 5 Gew.-%, jeweils bezogen auf das Gesamtgewicht der Zusammensetzung, vorliegt oder vorliegen.

Fig. 1

O/W/O-Emulsion

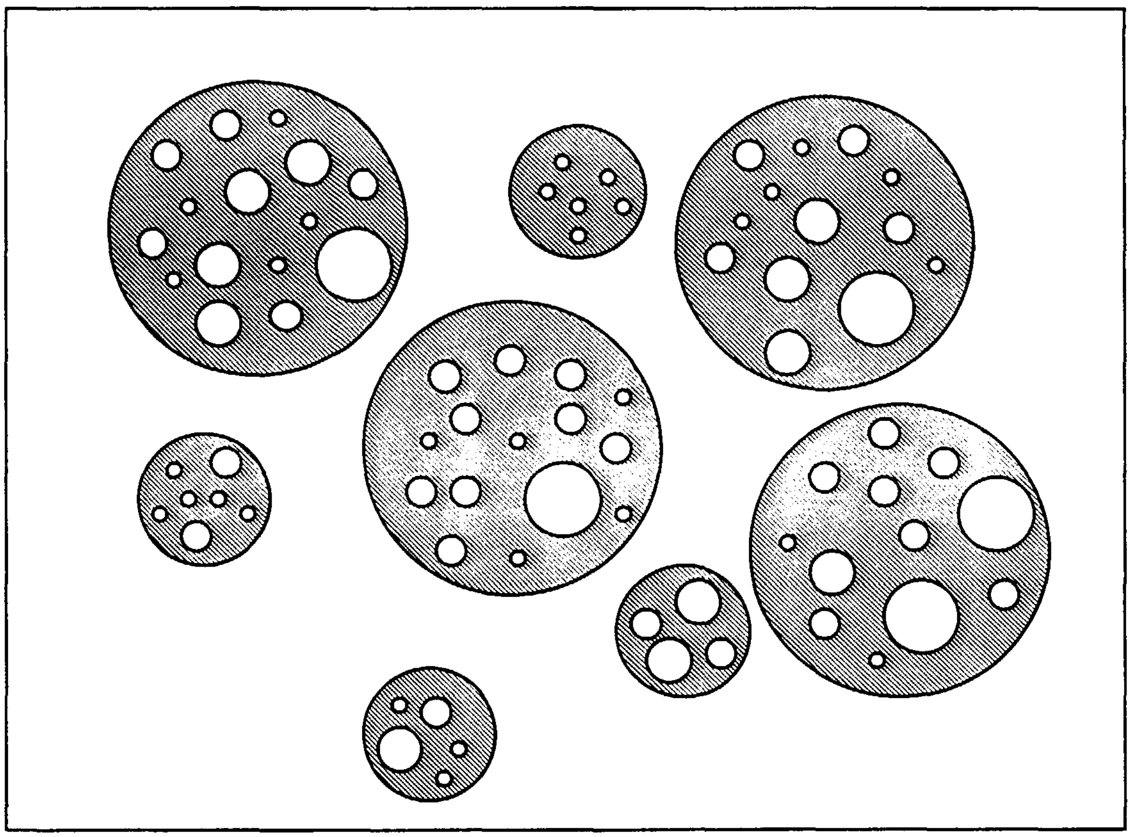

Fig. 2

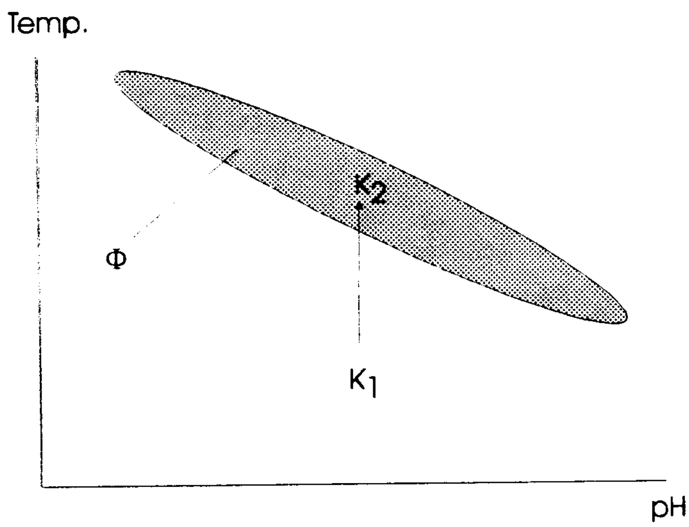

Europäisches Patentamt

# EUROPÄISCHER RECHERCHENBERICHT

Nummer der Anmeldung
EP 96 11 9424

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
| A | DE 43 43 833 A (BEIERSDORF AG)<br>* Ansprüche 1-13 *<br>--- | | A61K7/00<br>B01F17/00 |
| A | WO 92 18227 A (BEIERSDORF AG)<br>* Ansprüche 1-15 *<br>----- | | |
| | | | RECHERCHIERTE SACHGEBIETE (Int.Cl.6) |
| | | | A61K<br>B01F |

Der vorliegende Recherchenbericht wurde für alle Patentansprüche erstellt

| Recherchenort | Abschlußdatum der Recherche | Prüfer |
|---|---|---|
| DEN HAAG | 21.März 1997 | Fouquier, J-P |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)